# FASCICULE DE BREVET EUROPEEN

(11) **EP 2 310 031 B1**
(45) Date de publication et mention de la délivrance du brevet: **20.11.2013**
(21) Numéro de dépôt: 09766021.1
(22) Date de dépôt: 18.06.2009
(51) Int. Cl.: A61K 38/05, A61K 31/7004, A61K 8/97, A61K 8/64, A61K 8/60, A61K 8/49, A61Q 19/08, A61P 17/00

(54) **ASSOCIATION DE TILIROSIDE ET DE LYSINE-VALINE 5 DIAMINOHYDROXYBUTYRATE**
KOMBINATION AUS EINEM TILIROSID LYSIN-VALIN 5 DIAMINOHYDROXYBUTYRAT
COMBINATION OF A TILIROSIDE AND LYSINE-VALINE 5 DIAMINOHYDROXYBUTYRATE

(30) Priorité: 19.06.2008 FR 0803425
(43) Date de publication de la demande: 20.04.2011
(73) Titulaire: Laboratoires Clarins, 95304 Cergy Pontoise Cedex (FR)
(72) Inventeur: COURTIN, Olivier, F-92200 Neuilly Sur Seine (FR)
(74) Mandataire: Mena, Sandra
(86) Numéro de dépôt international: PCT/FR2009/000732
(87) Numéro de publication internationale: WO 2009/153459

(56) Documents cités:
- EP-A1- 1 393 733
- US-A1- 2004 081 675
- ANONYMOUS: "Syn -Tacks" INTERNET ARTICLE, [Online] décembre 2006 (2006-12), XP002509944 Extrait de l'Internet: URL:http://www.pentapharm.com/graphics/Pen tapharm/download/cosmetics/Anti-wrinkle_Sk in-Regeneration_Functional-Support/801045_ SYN-TACKS_Factsheet5904.pdf> [extrait le 2009-01-13]
- ANONYMOUS: "Syntacks Active Potion" INTERNET ARTICLE, [Online] 2006, pages 1-3, XP002509945 Extrait de l'Internet: URL:http://www.platinumskincare.com/syntac ksactive.aspx> [extrait le 2009-01-13]
- RECIO M C ET AL: "Studies on the radical scavenger and anti-inflammatory activities of tiliroside" IMFLAMMATION RESEARCH, BIRKHAEUSER VERLAG, BASEL, vol. 50, no. Supplement 3, 1 septembre 2001 (2001-09-01), page S205, XP008025202 ISSN: 1023-3830

## Description

La présente invention concerne une association comprenant au moins un bioflavonoïde, en particulier le tiliroside et au moins un peptide, en particulier le palmitoyl Lysine-Valine-5 diaminohydroxybutyrate. La présente invention concerne également une composition cosmétique ou dermatologique comprenant cette association, pouvant être utilisée pour prévenir, pour retarder ou pour lutter contre le vieillissement de la peau et/ou l'apparition des signes de vieillissement de la peau. En particulier, cette composition accélère et/ou stimule la synthèse du collagène de type I et favorise également la cicatrisation de la peau.

La présente invention a également pour objet un procédé de traitement cosmétique de la peau.

Chez l'homme, sur le plan anatomique, la peau comprend deux parties principales. La partie superficielle mince qui s'appelle épiderme est rattachée à une partie interne plus épaisse, le derme.

L'épiderme (étymologiquement formé en grec des mots *epi,* sur et *derma,* peau) désigne le tissu de nature épithéliale qui recouvre le derme. L'épiderme est composé principalement de trois types de cellules : les kératinocytes, les mélanocytes et les cellules de Langerhans. L'épiderme n'est irrigué par aucun vaisseau sanguin. Les cellules qui le composent sont alimentées par diffusion depuis le derme. Il contient, en revanche, de nombreuses terminaisons nerveuses.

Le derme, clairement séparé de l'épiderme par la « jonction dermo-épidermique », se poursuit en profondeur sans limite franche par l'hypoderme.

Le derme est constitué de différents types cellulaires et en particulier des fibroblastes, responsables de la synthèse et de l'entretien du matériel extracellulaire. Ce sont des cellules d'origine mésenchymateuse, qui synthétisent le collagène, l'élastine, la substance fondamentale et les glycoprotéines de structure. Leur activité est intense au cours des phénomènes de cicatrisation.

Le derme est constitué d'un tissu conjonctif qui associe des fibres de collagène, des fibres élastiques et diverses cellules baignant dans une substance fondamentale amorphe. Il contient également les annexes cutanées, qui sont les follicules pilo-sébacés et les glandes sudorales, ainsi que des vaisseaux et des nerfs. Par ses fibres et sa substance fondamentale, le derme contribue tout d'abord à donner à la peau ses propriétés mécaniques : élasticité, résistance au choc, etc.

Les fibres du derme comprennent les fibres de collagène et les fibres élastiques. Elles représentent quantitativement les protéines de structure les plus importantes du derme, soit respectivement 75% et 5% de leur poids sec. Leur proportion relative et leur disposition sont différentes selon les zones superficielles ou profondes du derme.

Les collagènes forment une très grande famille. Ce sont des molécules de la matrice extracellulaire composées de trois chaînes polypeptidiques portant la répétition de 3 acides aminés :
-Gly-X-Y, où X et Y sont souvent des prolines et hydroxyprolines.

Les «fibres de collagène» du derme sont constituées respectivement de collagène I et de collagène III, autour d'un axe composé de collagène V. Ces collagènes appartiennent au groupe des collagènes fibrillaires. Chez l'adulte, le collagène I est, en moyenne, six fois plus abondant que le collagène III.

La proportion de collagène I augmente quand on se rapproche de l'hypoderme.

Le rapport collagène I / collagène III diminue au cours du vieillissement. On assiste à une augmentation exponentielle de pontages chimiques entre les fibres de collagène dus à la réaction de glycation non enzymatique de Maillard. Ce pontage chimique du collagène âgé aboutit à une rigidification croissante des fibres qui les rend plus résistantes à l'attaque par les collagénases et par les radicaux libres. Sa dégradation et son renouvellement sont ainsi ralentis.

Le fibroblaste est une cellule clef du tissu conjonctif qui intervient dans la formation et la stabilisation des fibres élastiques, mais aussi dans leur dystrophie et leur lyse. Lors du vieillissement, le fibroblaste diminue son activité et cette cellule au repos est souvent appelée fibrocyte. Il devient globuleux, avec diminution de son cytoplasme et raréfaction de son réticulum endoplasmique dont les vésicules sont très dispersées. Le cytosquelette prend un aspect fasciculé. Cette cellule n'est plus en contact avec le collagène.

On comprend à la lecture de ce qui précède l'importance du collagène dans la structure du derme et par conséquent, la nécessité de stimuler et accélérer sa synthèse afin de prévenir, de retarder ou de lutter contre le vieillissement cutané, et afin de favoriser les processus de cicatrisation.

Le but de la présente invention est de mettre à disposition un produit qui permet de stimuler et/ou accélérer la synthèse du collagène de type I chez un mammifère et en particulier chez l'homme.

Un autre but de la présente invention est de mettre à disposition un produit qui permet de traiter de manière préventive ou curative chez un mammifère en général, et en particulier chez l'homme le vieillissement de la peau et/ou l'apparition des signes du vieillissement de la peau.

Récemment on a assisté, pour la prévention ou le traitement du vieillissement cutané, au développement de techniques dermatologiques à visée esthétique telles que les peelings, les lasers, les lampes flash pulsée, les traitements par radiofréquences, les traitements par LED etc.

Ces techniques de traitement cosmétiques ou dermatologiques peuvent induire une abrasion cutanée qui nécessite ensuite une cicatrisation et/ou une néosynthèse de fibres pour des effets restructurants dermiques à visée anti-âge.

Un but de la présente invention est la mise à disposition d'un produit favorisant la cicatrisation de la peau d'un mammifère et en particulier d'un être humain.

La présente invention a également pour but la mise à disposition d'un produit qui permet chez un mammifère et en particulier chez un humain, lors d'un traitement cosmétique et/ou dermatologique sélectionné parmi les traitements cités ci-dessus, de favoriser la néosynthèse du collagène et d'optimiser le remodelage cutané.

Encore un autre but de la présente invention est la mise à disposition d'un procédé de traitement cosmétique de la peau pour prévenir, pour retarder ou lutter contre l'apparition des signes de vieillissement et/ou les atteintes cutanées liées à l'âge et/ou pour favoriser la néosynthèse du collagène et/ou pour optimiser le remodelage cutané lors d'un traitement dermatologique sélectionné parmi un traitement au laser, un traitement par lampe flash pulsée, un traitement par des radiofréquences, un traitement par LED, un peeling et une microdermabrasion.

Pour atteindre ces buts, la Demanderesse a découvert une association comprenant au moins un bioflavonoïde, plus particulièrement le tiliroside, et au moins un peptide, le palmitoyl Lysine-Valine-5 diaminohydroxybutyrate ou Palm-Lys-Val-Dab-OH, dotée de propriétés avantageuses.

Le peptide Palm-Lys-Val-Dab-OH est connu dans l'art antérieur pour ses actions biologiques telles que la stimulation de la synthèse de la laminine V et la stimulation de la synthèse du collagène I. Il est décrit pour ses applications en tant qu'actif anti-âge, anti-rides et réparateur.

Le tiliroside a déjà été décrit dans l'art antérieur comme un actif anti-âge pour les peaux sensibles et comme un agent protecteur contre le stress oxydant et les procédés de micro inflammation. Il est également connu pour ses propriétés anti-allergiques, anti-élastase et anti-collagénase.

Ainsi, dans le document US 2004/0081675 le tiliroside est décrit et revendiqué en tant que filtre UV, agent antioxydant, agent anti-radicaux libres, actif contre le stress oxydant, anti-âge, anti-allergique, anti-inflammatoire et stabilisateur de filtre UV.

Le document EP 1 393 733 décrit le tiliroside pour le traitement de l'eczéma atopique. De plus, l'utilisation d'une composition comprenant du tiliroside pour traiter les pathologies affectant le collagène est mentionnée.

Toutefois aucun de ces documents ne mentionne ou ne suggère que le tiliroside aurait une activité stimulante de la synthèse du collagène I.

L'association comprenant le tiliroside et le palmitoyl Lysine-Valine-5 diaminohydroxybutyrate ainsi que son activité stimulatrice de la synthèse du collagène, n'ont jamais été décrites.

En effet, de façon surprenante, il a été découvert par la demanderesse que l'association du tiliroside et du palmitoyl Lysine-Valine-5 diaminohydroxybutyrate présente une activité stimulante de la synthèse du collagène I, et que cette activité est très supérieure à la somme des effets de chacun de ces actifs pris séparément.

Une propriété remarquable de l'association de la présente invention est qu'elle présente des effets dans des proportions plus importantes que celles raisonnablement attendues de la simple adition des effets de chacun de ces composés pris séparément.

Un avantage de cette propriété est de permettre une utilisation dans une composition cosmétique ou dermatologique d'une quantité de chacun de ces produits inférieure à ce qu'il est généralement admis d'utiliser.

Ainsi, la présente invention a pour objet une association comprenant au moins un bioflavonoïde, le tiliroside et au moins un peptide, le palmitoyl Lysine-Valine-5 diaminohydroxybutyrate.

Le tiliroside (C₃₀H₂₆O₇) est un composé connu et disponible commercialement. Pour la mise en oeuvre de la présente invention, il peut être utilisé pur ou sous forme d'un extrait végétal. Il est commercialisé par Merck KgaA sous forme purifiée à plus de 95%. Le document WO 2006/099930 décrit la préparation du tiliroside à partir de plantes.

Le palmitoyl Lysine-Valine-5 diaminohydroxybutyrate est commercialisé par la société Pentapharm en solution à 0,2% dans un solvant hydroglycériné.

De préférence, le pourcentage en poids de tiliroside (T) par rapport au palmitoyl Lysine-Valine-5 diaminohydroxybutyrate (P), T/P est compris entre 0,1 et 1000, avantageusement entre 0,25 et 200.

La présente invention a également pour objet une composition cosmétique et/ou dermatologique comprenant une association telle que décrite ci-dessus dans un support cosmétiquement ou dermatologiquement acceptable.

Les supports cosmétiquement acceptables, c'est-à-dire des supports compatibles avec la peau se présentent sous toutes les formes connues par l'Homme du métier et normalement utilisées pour une application topique, notamment sous la forme d'une émulsion huile-dans-eau ou eau-dans-huile, d'une solution aqueuse, huileuse ou hydroalcoolique, d'un gel aqueux ou huileux, d'un produit anhydride liquide, pâteux ou solide, d'une dispersion d'huile dans une phase aqueuse.

La composition de l'invention peut également contenir des adjuvants habituellement utilisés dans les domaines cosmétique et dermatologique tels que les gélifiants hydrophiles ou lypophiles, les actifs hydrophiles ou lipophiles, les conserveurs, les antioxydants, les solvants, les parfums, les filtres, les charges, les pigments, les agents chélateurs et les colorants.

La composition de l'invention peut être sous forme de crèmes, de gels, de sérums, de lotions, de laits ou d'huiles. Elle peut, le cas échéant être appliquée sur la peau sous forme d'aérosol. Elle peut se présenter également sous forme solide, par exemple sous forme de stick.

De préférence, cette composition comprend 0,01 à 10% en poids de tiliroside et 0,0001 à 1% en poids de palmitoyl Lysine-Valine-5 diaminohydroxybutyrate. Avantageusement, la composition comprend 0,01 à 1% en poids de tiliroside et 0,005 à 0,5% en poids de palmitoyl Lysine-Valine-5 diaminohydroxybutyrate. De manière encore plus avantageuse, la composition comprend 0,05 à 0,5% en poids de tiliroside et 0,001 à 0, 1 % en poids de palmitoyl Lysine-Valine-5 diaminohydroxybutyrate.

La composition selon la présente invention peut être utilisée pour stimuler et/ou accélérer la synthèse du collagène de type I. Cette stimulation et/ou accélération de la synthèse du collagène I est due à l'effet synergique de l'association du tiliroside et du palmitoyl Lysine-Valine-5 diaminohydroxybutyrate.

Cette propriété de la composition de la présente invention peut être utilisée dans toutes les circonstances où une néo synthèse de collagène est souhaitée.

Par conséquent, la composition selon la présente invention peut être utilisée pour prévenir, pour retarder ou pour lutter contre le vieillissement de la peau et/ou l'apparition des signes du vieillissement de la peau

Dans le contexte de l'invention, par signes du vieillissement de la peau on entend toutes les modifications de l'aspect extérieur de la peau dues au vieillissement chrono biologique et photo induit. Ces signes du vieillissement cutané sont par exemple l'apparition des rides et ridules, du relâchement cutané, d'une peau flétrie, d'une peau amincie, et d'une peau terne et/ou sans éclat, le manque d'élasticité et/ou de tonus de la peau. Parmi les signes du vieillissement cutané peuvent être mentionnés également toutes les modifications internes de la peau qui ne se traduisent pas systématiquement par un aspect extérieur modifié, par exemple toutes les dégradations internes de la peau et en particulier la dégradation du collagène.

En outre, l'apparition des vergetures due à la rupture des fibres de collagène et des fibres élastiques suite notamment à un étirement trop rapide et trop brutal de la peau, peut être également prévenue et/ou traitée par la composition de l'invention.

Les vergetures apparaissent fréquemment au cours d'une grossesse ou à la suite d'un changement de poids important ou même d'une croissance rapide.

Certains traitements cosmétiques et/ou dermatologiques à visée esthétique peuvent induire une abrasion cutanée nécessitant une cicatrisation et/ou une néosynthèse du collagène pour optimiser le remodelage cutané. Parmi ces traitements on peut citer un traitement aux lasers, un traitement par lampe flash pulsée, un traitement par des radiofréquences, un traitement par une diode électroluminescente LED (Light Emitted Diod), un peeling et une microdermabrasion.

Les traitements par des lasers dermatologiques ou cosmétiques ont pour objectif la prévention et le traitement du vieillissement cutané.

Généralement, différents types de lasers sont utilisés en fonction du but à atteindre. Quel que soit le type de laser utilisé, un effet bénéfique sur le remodelage cutané est observé.

Les lasers ablatifs ont pour principe de détruire de très minces couches de peau. Lors du traitement, la lumière laser volatilise l'épiderme et la partie la plus superficielle du derme. Ces deux épaisseurs sont ainsi éliminées. Dans le derme, ce sont principalement les fibres d'élastine altérées qui sont éliminées, mais il y a également un effet thermique qui conduit à la formation des nouvelles fibres de collagène.

Les lasers utilisés ici sont des lasers au CO₂ et/ou à l'erbium.

Le laser CO₂ (10 600 nm) utilisé en mode continu induit une abrasion cutanée avec coagulation et s'adresse préférentiellement à un photovieillissement marqué. La cicatrisation demande environ 7 jours.

Le laser Er- Yag pulsé (2 950 nm), purement ablatif (sans coagulation, d'où induction de saignements), induit un resurfaçage plus léger et s'adresse à un photovieillissement moins marqué. La cicatrisation est plus rapide (éviction de 7 jours environ).

Les lasers de remodelage et les lampes de rejuvénation, donnent des résultats plus modestes et pratiquement sans effets secondaires.

Sans entraîner de dommages épidermiques, les lasers de remodelage induisent une néo synthèse de collagène. Ils améliorent la tonicité et la texture de la peau et lissent le relief (ridules).

On utilise des dispositifs dont la longueur d'onde est préférentiellement absorbée soit par l'eau du derme, soit par les vaisseaux superficiels du derme. On peut utiliser des lasers émettant dans l'infra rouge (1064, 1320, 1450, 1540 nm), dans le visible (laser pulsé à colorant, laser KTP) et des lampes pulsées.

Une nouvelle technique, le relissage fractionnel (photothermolyse fractionnelle) peut combiner les avantages des deux méthodes précédentes et de nouvelles techniques agissant plus spécifiquement sur le relâchement cutané. Cette technique est basée sur un laser (1500 mn) ne balayant pas toute la surface mais réalisant des impacts de 100 microns de diamètre. A chaque séance, 20 % du derme est traité. La cicatrisation est rapide (éryhème modéré associé à un aspect pseudo bronzé).

Les traitements radiofréquence RF (douloureux) et l'appareil à lumière infrarouge de 1100 à 1800 nm sont utilisés pour prévenir et/ou traiter le relâchement cutané.

Le peeling est une autre technique non chirurgicale qui permet d'éliminer une épaisseur cutanée précise et contrôlée, d'induire une régénération saine de la couche détruite et une stimulation anabolique des couches sous-jacentes. Les peelings sont classés en légers, moyens et profonds en fonction de la couche cutanée détruite. Les complications dues au peeling sont d'autant plus graves que le peeling est profond.

La microdermabrasion comprend la projection de cristaux d'hydroxyde d'alumine et l'aspiration. On peut jouer sur la puissance de l'aspiration, la vitesse de projection et le nombre de passages sur la même zone pour moduler l'abrasion qui peut être épidermique (exfoliation du stratum corneum), dermique superficielle (avec ou sans saignement), dermique (avec saignement).

Il est évident, de ce qui est décrit précédemment que toutes ces techniques à visée cosmétique et/ou dermatologique peuvent provoquer à différents dégrés une abrasion cutanée qui par la suite, nécessite une cicatrisation.

La composition selon la présente invention peut être utilisée pour stimuler et/ou favoriser la cicatrisation.

En particulier elle peut être utilisée pour favoriser ou stimuler la cicatrisation après un traitement cosmétique ou dermatologique tel que ceux décrits ci-dessus. Mais elle peut aussi être utilisée pour favoriser ou stimuler la cicatrisation d'une lésion due à une blessure ou à un acte chirurgical.

La composition objet de l'invention peut être également utilisée pour favoriser la néosynthèse du collagène et pour optimiser le remodelage cutané lors d'un traitement dermatologique ou cosmétique sélectionné parmi un traitement au laser, un traitement par lampe flash pulsée, un traitement par des radiofréquences, un traitement par LED, un peeling et une microdermabrasion.

En particulier, cette composition peut être utilisée en tant que produit cicatrisant pour accompagner des traitements tels que les traitements au laser, le peeling, la microdermabrasion etc. Dans ce cas, elle est appliquée sur la peau après le traitement cosmétique ou dermatologique et l'application est renouvelée de façon répétée avec une fréquence qui peut aller de 1 à 5 fois par jour et jusqu'à l'obtention d'un résultat satisfaisant.

La composition selon la présente invention peut être également utilisée pendant un des traitements mentionnés ci-dessus sur les zones traitées, pour optimiser les performances des traitements (par exemple : réjuvénation). La composition de l'invention possède un effet potentialisateur qui renforce l'action du traitement dermatologique ou cosmétique. Dans ce cas, la composition est appliquée sur la zone cutanée à traiter peu avant (quelques minutes ou quelques heure) le début de chaque acte du traitement cosmétique ou dermatologique.

La composition de la présente invention peut être aussi utilisée en alternance avec chaque acte et/ou après le traitement pour optimiser l'efficacité des traitements esthétiques. La fréquence et la durée du traitement par cette composition sont adaptées en fonction de l'individu et du traitement cosmétique et/ou dermatologique appliqué.

En outre, la composition de l'invention peut être utilisée au quotidien en tant que produit anti-âge ou favorisant la cicatrisation.

La présente invention a également pour objet un procédé de traitement cosmétique de la peau pour prévenir, pour retarder ou pour lutter contre les atteintes cutanées liées à l'âge et/ou l'apparition des signes de vieillissement cutané, pour favoriser la cicatrisation et/ou la néosynthèse du collagène et pour optimiser le remodelage cutané lors d'un traitement dermatologique sélectionné parmi un traitement au laser, un traitement par lampe flash pulsée, un traitement par des radiofréquences, un traitement par LED, un peeling et une microdermabrasion, ledit procédé comprenant l'application sur la peau de la composition faisant objet de la présente invention. Cette application peut être faite avant le traitement dermatologique ou cosmétique, elle peut intervenir entre deux actes de ce traitement ou elle peut être postérieure à ce traitement. Cette application sur les zones cutanées concernées peut être répétée avec une fréquence et pour une durée que l'Homme du métier sait adapter en fonction de l'individu et du traitement dermatologique ou cosmétique associé.

Quel que soit l'objectif, on peut proposer une application quotidienne, une ou deux fois par jour, pour une durée de quelques jours à plusieurs mois.

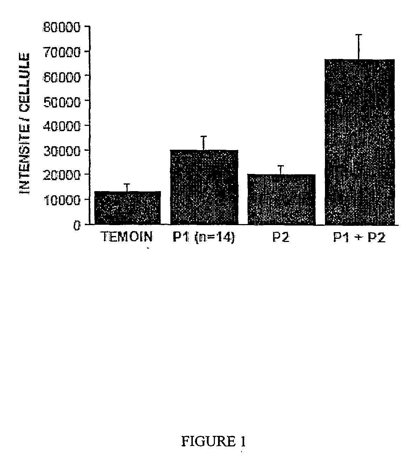

L'invention sera mieux comprise, et d'autres caractéristiques de celle-ci apparaîtront plus clairement à la lecture de l'exemple qui suit et qui fait référence à la figure 1 qui représente l'effet synergique de l'association du tiliroside et du palmitoyl Lysine-Valine-5 diaminohydroxybutyrate sur la synthèse du collagène de type I dans des fibroblastes humains normaux adultes.

L'invention est définie dans les revendications.

### EXEMPLE 1

En visualisant l'immuno-localisation du collagène de type I dans des fibroblastes humains normaux adultes, on a pu démontrer l'effet synergique de l'association du tiliroside et du palmitoyl Lysine-Valine-5 diaminohydroxybutyrate sur la synthèse du collagène de type I, protéine majeure de la matrice extracellulaire du derme.

### Produit à l'essai :

Nous avons utilisé du palmitoyl Lysine-Valine-5 diaminohydroxybutyrate 2.10⁻³% (P1), du tiliroside 5.10⁻⁴ % (P2), l'association des deux actifs (P1*P2) ci-dessus et un témoin (T).

Les fibroblastes ont été ensemencés dans des plaques 6 puits avec lamelle de verre à raison de 70.000 cellules par boîte dans du milieu MEM (Minimum Essential Medium).

48h après, les cellules ont été traitées ou non (témoin) par le palmitoyl Lysine-Valine-5 diaminohydroxybutyrate ou par le tiliroside ou par l'association des deux actifs et incubées à l'étuve pendant 72 heures à 37°C, 5%CO₂.

Chaque tapis cellulaire a été ensuite rincé et fixé au méthanol (-20°C) avant de procéder à la révélation du collagène de type I par immunofluorescence.

### Immunolocalisation du collagène de type I

Les cellules sont incubées avec le premier anticorps anti-collagène I (*Sigma,* dilution 1 :100) (source : souris) puis avec l'anticorps secondaire anti-souris couplé au TRITC *(Abcys,* dilution 1 :100). Le temps d'incubation entre chaque anticorps est d' 1 heure. Les noyaux cellulaires sont révélés par un marquage au Dapi.

Les lamelles sont montées sur lame de verre puis observées au microscope à fluorescence (Nikon Eclipse 50i). Plusieurs champs de la population cellulaire sont pris en photo. Les images de fluorescence TRITC indiquent les régions de localisation du collagène de type I. Les images de fluorescence DAPI indiquent les noyaux cellulaires et donc le nombre de cellules par champs. Les clichés photographiques sont analysés par le logiciel d'analyse d'image Lucia.

### Analyse statistique et résultats

L'intensité de marquage du collagène a été observée sur 14 et 15 clichés photographiques selon les conditions.

Cette intensité de fluorescence est rapportée au nombre de cellule par cliché.

Les différences de moyenne entre les populations ont été étudiées par l'analyse de la variance (Anova, ANalysis Of Variance). Cette méthode utilise des mesures de variance afin de déterminer le caractère significatif, ou non, des différences de moyenne mesurées sur les populations.

Les résultats obtenus présentés sur la figure 1 montrent que le palmitoyl Lysine-Valine-5 diaminohydroxybutyrate entraîne une augmentation, statistiquement significative (p<0,0001), du collagène par les fibroblastes de peau humaine par rapport au témoin non traité.

Le tiliroside stimule d'une manière moins importante mais statistiquement significative (p=0,0015), l'expression du collagène de type I.

La présence simultanée du palmitoyl Lysine-Valine-5 diaminohydroxybutyrate et du tiliroside entraîne une stimulation plus importante de la synthèse du collagène de type I que chacun des constituants pris isolément (interaction significative p=0,0278).

Cet effet synergique entraîne une surexpression du collagène de type I.

### EXEMPLES DE COMPOSITIONS

Le peptide utilisé dans les exemples de compositions qui suivent est le palmitoyl Lysine-Valine-5 diaminohydroxybutyrate, sous la forme commerciale fournie par la société Pentapharm en solution à 0,2% dans un solvant hydroglycériné.

Les pourcentages sont des pourcentages en poids par rapport au poids total de la composition.

**EMULSION E/H CICATRISANTE**

| | % |
|---|---|
| GLYCERINE | 5,00 |
| SULFATE DE MAGNESIUM | 0,70 |
| SULFATE DE ZINC | 0,35 |
| SULFATE DE CUIVRE | 0,10 |
| GLYCOLS | 5,00 |
| PEG 30 DIPOLYHYDROXYSTEARATE | 4,00 |
| TRIGLYCERIDES C16-C18 HYDROGENES | 5,00 |
| PEG-45/DODECYL GLYCOL COPOLYMER | 1,20 |
| TRIGLYCERIDES C8 C10 | 19,00 |
| HUILE DE VASELINE FLUIDE | 8,00 |
| CERAMIDE 3 | 0,10 |
| OXYDE DE ZINC | 4,00 |
| TILIROSIDE | 0,10 |
| ACETATE DE TOCOPHEROL | 0,20 |
| PEPTIDE | 1,00 |
| EAU DEMINERALISEE | Q.S.P 100 |

**GEL ANTI-AGE**

| | % |
|---|---|
| GELIFIANT POLYMERIQUE | 1,00 |
| GLYCOLS | 5,00 |
| TILIROSIDE | 0,10 |
| PEPTIDE | 1,00 |
| CHELATANT | 0,10 |
| CONSERVATEUR | 0,10 |
| EAU DEMINERALISEE | Q.S.P 100 |

**EMULSION H/E ANTI-AGE**

| | % |
|---|---|
| CETEARYL GLUCOSIDE | 3,50 |
| MONOSTEARATE DE GLYCEROL AE | 2,50 |
| TRIGLYCERIDES C8 C10 | 5,00 |
| BEURRE DE KARITE | 3,00 |
| CETEARYL OCTANOATE | 7,00 |
| ALCOOL CETYLIQUE | 0,30 |
| ALCOOL STEARYLIQUE | 0,30 |
| SILICONE | 0,50 |
| CONSERVATEUR | 0,10 |
| GLYCOLS | 3,00 |
| SILICONE VOLATIL | 5,00 |
| GLYCERINE | 5,00 |
| TILIROSIDE | 0,20 |
| PEPTIDE | 1,00 |
| ACETATE DE TOCOPHEROL | 0,20 |
| GELIFIANT | 0,30 |
| CHELATANT | 0,20 |
| COLORANT | 0,06 |
| SOUDE | Q.S. pH |
| EAU DEMINERALISEE | Q.S.P 100 |

## Revendications

1. Association comprenant du tiliroside et du palmitoyl Lysine-Valine-5 diaminohydroxybutyrate.

2. Association selon la revendication 1, dans laquelle le pourcentage en poids de tiliroside (T) par rapport au palmitoyl Lysine-Valine-5 diaminohydroxybutyrate (P) T/P est compris entre 0,1 et 1000, avantageusement entre 0,25 et 200.

3. Composition cosmétique et/ou dermatologique comprenant une association selon l'une quelconque des revendications 1 et 2 dans un support cosmétiquement ou dermatologiquement acceptable.

4. Composition cosmétologique et/ou dermatologique comprenant :
- 0,01 à 10% en poids de tiliroside, avantageusement de 0,05 à 0,5% ;
- 0,0001 à 1% en poids de palmitoyl Lysine-Palm-Lys-Valine-5 diaminohydroxybutyrate, avantageusement de 0,001 à 0,1%.

5. Composition selon l'une quelconque des revendications 3 et 4, ladite composition étant sous une forme sélectionnée parmi : une crème, un gel, un sérum, une lotion, un lait, et une huile.

6. Composition selon l'une quelconque des revendications 3 à 5 pour son utilisation pour stimuler et/ou accélérer la synthèse du collagène de type I.

7. Composition selon l'une quelconque des revendications 3 à 5 pour son utilisation pour prévenir, pour retarder ou pour lutter contre le vieillissement de la peau et/ou l'apparition des signes de vieillissement de la peau.

8. Composition selon la revendication 7, dans laquelle les signes de vieillissement de la peau sont sélectionnés parmi les rides, les ridules, un relâchement cutané, une peau flétrie, une peau amincie, et une peau terne et/ou sans éclat.

9. Composition selon l'une quelconque des revendications 3 à 5 pour son utilisation pour prévenir ou lutter contre les vergetures.

10. Composition selon l'une quelconque des revendications 3 à 5 pour son utilisation pour favoriser la cicatrisation.

11. Composition selon l'une quelconque des revendications 3 à 5 pour son utilisation pour favoriser la néosyntèse du collagène et pour optimiser le remodelage cutané lors d'un traitement dermatologique ou cosmétique sélectionné parmi : un traitement au laser, un traitement par lampe flash pulsée, un traitement par des radiofréquences, un traitement par LED, un peeling et une microdermabrasion.

12. Procédé de traitement cosmétique de la peau pour prévenir, pour retarder ou pour lutter contre les atteintes cutanées liées à l'âge et/ou l'apparition des signes du vieillissement et/ou pour favoriser la néosynthèse du collagène et/ou pour optimiser le remodelage cutané lors d'un traitement dermatologique sélectionné parmi un traitement au lasers, un traitement par lampe flash pulsée, un traitement par des radiofréquences, un traitement par LED, un peeling et une microdermabrasion, **caractérisé par le fait qu'**on applique sur la peau une composition telle que définie dans l'une quelconque des revendications 3 à 5.

## Patentansprüche

1. Kombination, umfassend Tilirosid und Palmitoyl-Lysin-Valin-5-Diaminohydroxybutyrat.

2. Kombination gemäß Anspruch 1, wobei der Gewichtsprozent-anteil von Tilirosid (T), bezogen auf Palmitoyl-Lysin-Valin-5-Diaminohydroxybutyrat (P), T/P zwischen 0,1 und 1000, vorteilhafterweise zwischen 0,25 und 200, liegt.

3. Kosmetische und/oder dermatologische Zusammensetzung, umfassend eine Kombination gemäß einem der Ansprüche 1 und 2 in einem kosmetisch oder dermatologisch annehmbaren Träger.

4. Kosmetologische und/oder dermatologische Zusammensetzung, umfassend:
- 0,01 bis 10 Gew.-% Tilirosid, vorteilhafterweise 0,05 bis 0,5%;
- 0,0001 bis 1 Gew.-% Palmitoyl-Lysin-Palm-Lys-Valin-5-Diaminohydroxybutyrat, vorteilhafterweise 0,001 bis 0,1%.

5. Zusammensetzung gemäß einem der Ansprüche 3 und 4, wobei die Zusammensetzung in einer Form ist, die ausgewählt ist aus: einer Creme, einem Gel, einem Serums., einer Lotion, einer Milch und einem Öl.

6. Zusammensetzung gemäß einem der Ansprüche 3 bis 5 zu ihrer Verwendung, um die Synthese von Kollagen Typ I zu stimulieren und/oder zu beschleunigen.

7. Zusammensetzung gemäß einem der Ansprüche 3 bis 5 zu ihrer Verwendung, um die Alterung der Haut und/oder das Auftreten vor Alterungszeichen der Haut zu verhindern, zu verzögern oder zu bekämpfen.

8. Zusammensetzung gemäß Anspruch 7, wobei die Alterszeichen der Haut ausgewählt sind aus Falten, Fältchen, Hauterschlaffung, einer welken Haut, einer dünneren Haut und einer fahlen Haut und/oder einer Haut ohne Glanz.

9. Zusammensetzung gemäß einem der Ansprüche 3 bis 5 zu ihrer Verwendung, um Schwangerschaftsstreifen zu verhindern oder zu bekämpfen.

10. Zusammensetzung gemäß einem der Ansprüche 3 bis 5 zu ihrer Verwendung, um die Wundhe-lung bzw. Narbenbildung zu begünstigen.

11. Zusammensetzung gemäß einem der Ansprüche 3 bis 5 zu ihrer Verwendung, um die Neusynthese von Kollagen zu begünstigen und die Hautremodulierung zu optimieren, und zwar während einer dermatologischen oder kosmetischen Behandlung, ausgewählt aus: einer Laserbehandlung, einer Behandlung mit einer gepulsten Blitzlampe, einer Behandlung durch Radiofrequenzen, einer Behandlung durch LED, einem Peeling und einer Mikrodermabrasion.

12. Kosmetisches Behandlungsverfahren für die Haut, um altersbedingte Hautschäden und/oder das Auftreten von Zeichen der Alterung zu verhindern, zu verzögern oder zu bekämpfen, und/oder um die Neusynthese von Kollagen zu begünstigen und/oder um die Hautremodulierung zu optimieren, und zwar während einer dermatologischen Behandlung, ausgewählt aus einer Behandlung mit Laser, einer Behandlung mit gepulster Blitzlampe, einer Behandlung durch Radiofrequenzen, einer Behandlung durch LED, einem Peeling und einer Mikrodermabrasion, **dadurch gekennzeichnet, dass** man eine Zusammensetzung, wie sie in einem der Ansprüche 3 bis 5 definiert ist, auf die Haut aufträgt.

## Claims

1. Association comprising tiliroside and palmitoyl lysine-valine-5 diaminohydroxybutyrate.

2. Association according to claim 1, wherein the percentage by weight of tiliroside (T) relative to the palmitoyl lysine-valine-5 diaminohydroxybutyrate (P), T/P, is between 0.1 and 1000, advantageously between 0.25 and 200.

3. Cosmetic and/or dermatological composition comprising an association according to either claim 1 or claim 2 in a cosmetically or dermatologically acceptable carrier.

4. Cosmetic and/or dermatological composition comprising:
- 0.01 to 10 % by weight of tiliroside, advantageously from 0.05 to 0.5 %;
- 0.0001 to 1 % by weight of palmitoyl lysine-palm-lys-valine-5 diaminohydroxybutyrate, advantageously from 0.001 to 0.1 %.

5. Composition according to either claim 3 or claim 4, said composition being in a form selected from: a cream, a gel, a serum, a lotion, a milk and an oil.

6. Composition according to any of claims 3 to 5 for use in stimulating and/or accelerating the synthesis of collagen type I.

7. Composition according to any of claims 3 to 5, for use in preventing, delaying or combatting skin aging and/or the appearance of the signs of skin aging.

8. Composition according to claim 7, wherein the signs of skin aging are selected from wrinkles, fine lines, sagging of the skin, weathered skin, thinned skin, and dull and/or lifeless skin.

9. Composition according to any of claims 3 to 5, for use in preventing or combatting stretch marks.

10. Composition according to any of claims 3 to 5, for use in promoting cicatrisation.

11. Composition according to any of claims 3 to 5, for use in promoting collagen neosynthesis and for optimising skin remodelling during a dermatological or cosmetic treatment selected from: a laser treatment, a pulsed flash lamp treatment, a radiofrequency treatment, an LED treatment, a peel and a microdermabrasion.

12. Method for cosmetically treating the skin for preventing, delaying or combatting at age-related skin damage, and/or the appearance of the signs of aging, and/or for promoting collagen neosynthesis and/or for optimising skin remodelling during a dermatological treatment selected from a laser treatment, a pulsed flash lamp treatment, a radiofrequency treatment, an LED treatment, a peel and a microdermabrasion, **characterised in that** a composition as defined in any of claims 3 to 5 is applied to the skin.
